(19) **Europäisches Patentamt**

**European Patent Office**

**Office européen des brevets**

(11) **EP 1 029 840 B1**

(12) **EUROPEAN PATENT SPECIFICATION**

(45) Date of publication and mention
of the grant of the patent:
**06.05.2004 Bulletin 2004/19**

(51) Int Cl.[7]: **C07C 17/386**, C07C 19/08

(21) Application number: **00112323.1**

(22) Date of filing: **12.07.1996**

(54) **Process for separating HFC-32 and HFC-125**

Verfahren zur Trennung von HFC-32 und HFC-125

Procédé pour la séparation de HFC-32 et HFC-125

(84) Designated Contracting States:
**DE ES FR GB IT NL**

(30) Priority: **14.07.1995 US 1156 P**

(43) Date of publication of application:
**23.08.2000 Bulletin 2000/34**

(62) Document number(s) of the earlier application(s) in
accordance with Art. 76 EPC:
**96923764.3 / 0 840 719**

(73) Proprietor: **E.I. DUPONT DE NEMOURS AND
COMPANY**
**Wilmington, Delaware 19898 (US)**

(72) Inventors:
• **Miller, Ralph Newton**
**Newark, DE 19711-2477 (US)**

• **Mahler, Barry Asher**
**Glen Mills, PA 19342-1258 (US)**
• **Nappa, Mario Joseph**
**Newark, DE 19711-3435 (US)**
• **Casey, Mark Andrew**
**Newark, DE 19711-3413 (US)**

(74) Representative: **KUHNEN & WACKER**
**Patent- und Rechtsanwalsbüro**
**Postfach 19 64**
**85319 Freising (DE)**

(56) References cited:
**US-A- 3 101 304**

Remarks:
The file contains technical information submitted
after the application was filed and not included in this
specification

**Description**

FIELD OF THE INVENTION

The instant invention relates to a process for separating HFC-32 and HFC-125 from a first mixture by using an extractant comprising methylene chloride, the process comprising the steps of: adding the extractant to the first mixture in order to form a second mixture, separating HFC-32 and HFC-125 in the second mixture by extractively distilling the second mixture in an extractive distillation zone of a distillation column and thereby recovering HFC-125 as an overhead product of the column and HFC-32 as a bottom product from the column.

BACKGROUND OF THE INVENTION

New regulations have been designed to protect the atmospheric ozone layer from possible damage by fully halogenated chlorofluorocarbons, also known as CFC's. Pentafluoroethane (CHF2-CF3 or HFC-125) is a useful non-chlorine containing fluorocarbon that is especially desirable for use as a refrigerant, blowing agent, propellant, fire extinguishing agent, sterilant carrier gas, among other uses.

Pentafluoroethane may be prepared by chlorofluorinating perchloroethylene (perclene or CCl2=CCl2) to produce a mixture comprising trichlorotrifluoroethane (CF2Cl-CFCl2 or CFC-113), dichlorotetrafluoroethane (CF2Cl-CF2Cl or CFC-114) and dichlorotrifluoroethane (CHCl2-CF3 or HCFC-123), thereafter removing trichlorotrifluoroethane, and; fluorinating the remaining mixture to produce a mixture comprising pentafluoroethane (HFC-125), chloropentafluoroethane (CF3-CF2C1 or CFC-115), and smaller amounts of other fluorinated compounds, e.g., hexafluoroethane (CF3-CF3 or FC-116). Such a chlorofluorinating method is described in U.S. Patent No. 3,755,477. Various other methods for making pentafluoroethane together with chloropentafluoroethane are known and described, for example, in U.S. Patent Nos. 3,258,500, 5,334,787 and 5,399,549, Japanese Patent Application Publication Nos. JP 03/099026, JP 04/029941, European Patent Application Publication No 0 506 525, and World Intellectual Property Organization Publication No. WO 91/05752.

Difluoromethane (CH2F2 or HFC-32) is another desirable non-chlorine containing fluorocarbon that is also valuable as a refrigerant among other uses. HFC-32 can be formed, for example, by the catalytic fluorination of dichloromethane (HCC-30 or methylene chloride) with HF. HFC-32 is known to form an azeotropic or azeotrope-like composition with HFC-125.

CROSS-REFERENCE TO RELATED NON-PROVISIONAL PATENTS AND PATENT APPLICATIONS

Copending and commonly assigned U.S. Patent Application Serial No. 08/192,663, filed on February 7, 1994 (corresponding to PCT Publication No. WO 95/21147), and entitled "Process for Separating Pentafluoroethane from a Mixture Comprising Halogenated Hydrocarbons & Chloropentafluoroethane" relates to an extractive distillation process to remove CFC-115 from HFC-125 by using at least one of hydrocarbon, hydrochlorocarbon and chlorocarbon as extractive agents.

Copending and commonly assigned U.S. Patent Application Serial No. 08/192,664 and 08/378,349, filed respectively on February 7, 1994 and February 1, 1995 (corresponding to PCT Publication No. WO 95/21148), and entitled "Process for Separating Pentafluoroethane from a Mixture Comprising Halogenated Hydrocarbons and Chloropentafluoroethane" relates to an extractive distillation process to remove CFC-115 from HFC-125 by using alcohol-containing extractive agents.

SUMMARY OF THE INVENTION

The instant invention solves problems associated with conventional processes by providing a process wherein methylene chloride is used as an extractant in an extractive distillation process for separating HFC-32 and HFC-125, with HFC-32 exiting the extractive distillation column bottoms with the methylene chloride and HFC-125 exiting the extractive distillation column overhead.

The present invention relates to a process for separating HFC-32 and HFC-125 from a first mixture by using an extractant comprising methylene chloride. The process comprises the steps of adding the extractant to the first mixture in order to form a second mixture, separating HFC-32 and HFC-125 in the second mixture by extractively distilling the second mixture in an extractive distillation zone of a distillation column and thereby recovering HFC-125 as an overhead product of the column while recovering HFC-32 from the column bottom.

HFC-32 and HFC-125 can be co-produced by contacting a mixture comprising a HFC-32 precursor such as methylene chloride (HCC-30 or CH2Cl2) and a HFC-125 precursor such as HCFC-123 (dichlorotrifluoroethane) and/or HCFC-124 (chlorotetrafluoroethane) with HF in the presence of a catalyst, e.g., CrCl3 on a carbon support or Cr2O3.

The following equations illustrate suitable processes for co-producing HFC-32 and HFC-125:

$$CH2Cl2 + CHCl2\text{-}CF3 + 4HF \rightarrow CH2F2 + CHF2\text{-}CF3 + 4HCl$$

and/or

$$CH2Cl2 + CHClF\text{-}CF3 + 3HF \rightarrow CH2F2 + CHF2\text{-}CF3 + 3HCl$$

and/or

$$CH2ClF + CHCl2\text{-}CF3 + 3\ HF \rightarrow CH2F2 + CHF2\text{-}CF3 + 3\ HCl$$

and/or

$$CH2ClF + CHClF\text{-}CF3 + 2\ HF \rightarrow CH2F2 + CHF2\text{-}CF3 + 2\ HCl$$

The reaction product contains predominant quantities of HFC-32 and HFC-125 along with one or more of the following. HCFC-31 (chlorofluoromethane) HCC-30 (dichloromethane or methylene chloride), CFC-114a (dichlorotetrafluoroethane), CFC-115 (chloropentafluoroethane), HCFC-124 (chlorotetrafluoroethane), HCFC-133a (chlorotrifluoroethane), HF (hydrogen fluoride), HCl (hydrogen chloride), among others. Impurities other than CFC-115 and hydrogen fluoride can be efficiently removed from the resulting HFC-125 containing products by conventional distillation or other processes known by one skilled in the art. For example, the CFC-115 and/or the hydrogen fluoride can be removed from the HFC-125 containing products by employing the process of the parent application EP 0 840 719 A1.

In this invention, methylene chloride is employed as an extractant in an extractive distillation for separating HFC-32 and HFC-125. This process permits HFC-125 to exit overhead from the distillation column, while allowing methylene chloride and HFC-32 to exit the column bottoms.

BRIEF DESCRIPTION OF THE DRAWINGS

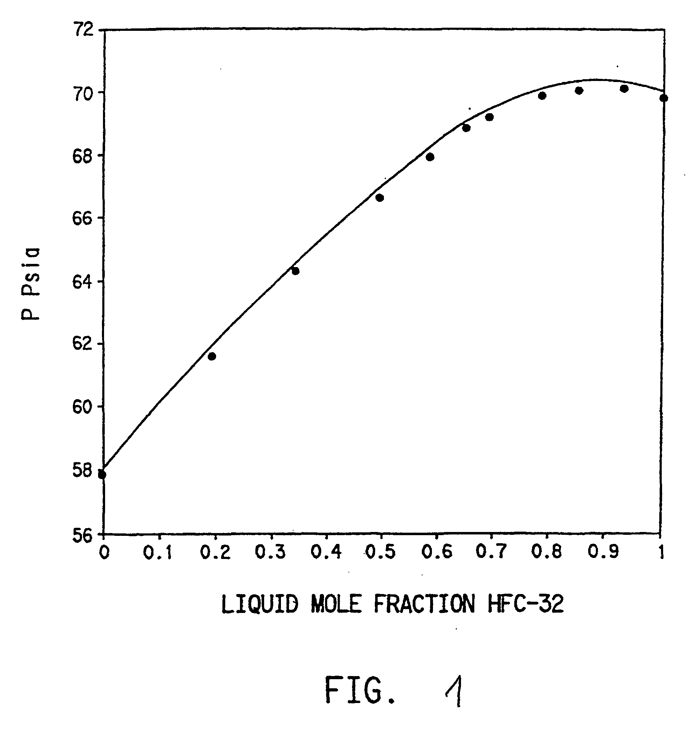

FIGURE 1 is a graphical representation at about -153 C of an azeotropic and azeotrope-like composition formed between HFC-32 and HFC-125.

DETAILED DESCRIPTION

The pentafluoroethane (HFC-125) and difluoromethane (HFC-32) which are the primary constituents of a first mixture, in their separated and pure state have boiling points, respectively, of about -48.5 and -51,7° degrees C. The relative volatility at atmospheric pressure of HFC-125/HFC-32 was found to be 1.0 as 90% purity of HFC-32 was approached (US 4 978 467; Table 1, col. 5). These data indicate that using conventional distillation processes will not result in the separation of a substantially pure compound.

To determine the relative volatility of HFC-125 and HFC-32 the so-called PTx Method was used. In this procedure, the total absolute pressure in a cell of known volume is measured at a constant temperature for various known binary compositions. Use of the PTx Method is described in greater detail in "Phase Equilibrium in Process Design", Wiley-Interscience Publisher, 1970, written by Harold R. Null, on pages 124 to 126; the entire disclosure of which is hereby incorporated by reference.

These measurements can be correlated to equilibrium vapor and liquid compositions in the cell by using an activity coefficient equation model, such as the Non-Random, Two-Liquid (NRTL) equation, to represent liquid phase non-idealities. Use of an activity coefficient equation, such as the NRTL equation, is described in greater detail in "The Properties of Gases and Liquids", 4th edition, publisher McGraw Hill, written by Reid, Prausnitz and Poling, on pages 241 to 387; and in "Phase Equilibria in Chemical Engineering", published by Butterworth Publishers, 1985, written by Stanley M. Walas, pages 165 to 244; the entire disclosure of each of the previously identified references is hereby incorporated by reference.

Finding the type of compounds which form azeotropic or azeotrope-like compositions, and finding the pair of compounds which specifically will form the lowest-boiling azeotrope at a particular temperature and/or pressure cannot be predicted, but must be identified by experiment (i.e., by measuring binary vapor-liquid equilibria data). Literature values by different investigators on the boiling points or vapor pressures of compounds and/or azeotropes at a variety of conditions may not provide consistent information for establishing which compounds or azeotropic or azeotrope-like compositions are lowest boiling under a single set of conditions, since the azeotropic or azeotrope-like compositions frequently change with pressure and temperature.

By "azeotrope" or "azeotropic" composition is meant a constant-boiling liquid admixture of two or more substances that behaves as a single substance. One way to characterize an azeotrope or azeotropic composition or mixture is that the vapor produced by partial evaporation or distillation of the liquid has the same composition as the liquid from which it was evaporated or distilled, e.g., the admixture distills/refluxes without compositional change. Constant boiling compositions are characterized as azeotropic because they exhibit either a maximum or minimum boiling point, as compared with that of the non-azeotropic mixture of the same components. An azeotropic composition can also be characterized as the maximum or minimum vapor pressure for a mixture at a given temperature when plotted as a function of mole fraction.

By "azeotrope-like" composition is meant a constant boiling, or substantially constant boiling admixture of two or more substances that behaves as a single substance. One way to characterize an azeotrope-like composition is that the vapor produced by partial evaporation or distillation of the liquid has substantially the same composition as the liquid from which it was evaporated or distilled, e.g., the admixture distills/refluxes without substantial compositional change. An azeotrope-like composition can also be characterized by the area, which is shown by plotting vapor pressure at a given temperature as a function of mole fraction, that is adjacent to the maximum or minimum vapor pressure.

Typically, a composition is azeotrope-like, if, after 50 weight percent of the composition is removed such as by evaporation or boiling off, the change between the original composition and the composition remaining is less than about 6 %, and normally less than about 3 % relative to the original composition.

By "effective amount" is intended to refer to the amount of each component of the inventive compositions which, when combined, results in the formation of an azeotropic or azeotrope-like composition. This definition includes the amount of each component, which amounts may vary depending on the pressure applied to the composition so long as the azeotropic or azeotropic-like compositions continue to exist at the different pressures, but with possible different boiling points. Effective amount also includes the amounts, such as may be expressed in weight percentages, of each component of the compositions of the instant invention which form azeotropic or azeotrope-like compositions at temperatures or pressures other than as described herein. Azeotropic or azeotrope-like compositions consist essentially of effective amounts of HFC-32 and HFC-125 such that after about 50 weight percent of an original composition is evaporated or boiled off to produce a remaining composition, the change between the original composition and the remaining composition is typically no more than about 6% and normally no more than about 3% or less relative to the original composition.

It is possible to characterize, in effect, a constant boiling admixture which may appear under many guises, depending upon the conditions chosen, by any of several criteria:

* The composition can be defined as an azeotrope of HFC-125 ("C") and HFC-32 ("D"), because the term "azeotrope" is at once both definitive and limitative, and requires effective amounts of C,D for this unique composition of matter which can be a constant boiling composition.

* It is well known by those skilled in the art, that, at different pressures, the composition of a given azeotrope will vary at least to some degree, and changes in pressure will also change, at least to some degree, the boiling point temperature. Thus, an azeotrope of HFC-125 ("C") and HFC-32 ("D") represents a unique type of relationship but with a variable composition which depends on temperature and/or pressure. Therefore, compositional ranges, rather than fixed compositions, are often used to define azeotropes.

* The composition can be defined as a particular weight percent relationship or mole percent relationship of HFC-125 ("C") and HFC-32 ("D"), which recognizing that such specific values point out only one particular relationship and that, in actuality, a series of such relationships, represented by C,D actually exist for a given azeotrope, varied by the influence of pressure.

* An azeotrope of HFC-125 ("C") and HFC-32 ("D"), can be characterized by defining the compositions as an azeotrope characterized by a boiling point at a given pressure, thus given identifying characteristics without unduly limiting the scope of the invention by a specific numerical composition, which is limited by and is only as accurate as the analytical equipment available.

The azeotrope or azeotrope-like compositions of the present invention may be formed by operating a conventional distillation apparatus, when practicing the inventive extractive distillation apparatus, and by combining effective amounts of the components by any convenient method including mixing, combining, among others. For best results, a preferred method is to weigh the desired component amounts, and thereafter combine them in an appropriate container.

Moreover, the HFC-125/HFC-32 azeotrope can also be formed under a variety of temperatures and pressures.

At a temperature of about -15.3 °C, an azeotrope can form consisting essentially of about 9.1 mole % HFC-125 and about 90.9 mole % HFC-32 having a pressure of about 484,7 kPa (70.3 psia). Referring now to Figure 1, Figure 1 illustrates graphically the results of measurements made at -15.3 °C showing formation of an azeotropic or azeotrope-like composition consisting essentially of HFC-32 and HFC-125, as indicated by mixture of about 90.9 moles % HFC-32 and about 9.1 mole % HFC-125 having a higher vapor pressure than either pure component or other HFC-32/HFC-125 mixtures at this temperature, with the composition of the vapor space in the maximum pressure region being that of the azeotrope at the specific temperature and pressure. At about 44.0 °C., we found an azeotrope forms consisting essentially of about 96.0 mole % HFC-32 and 4.0 mole % HFC-125 having a pressure of about 2799.3 kPa (406 psia). Based upon these results and the previously described NRTL references, we determined that an azeotrope can form at a temperature of about -50 °C consisting essentially of about 84.9 mole % HFC-32 and about 15.1 mole % HFC-125 having a pressure of about 111.7 kPa (16.2 psia).

As discussed previously, mixtures of HFC-125 and HFC-32 that are co-produced in accordance with the instant invention or obtained from any other suitable source are difficult to separate due to the formation of an HFC-125/HFC-32 azeotrope. When it is desired that either HFC-125 or HFC-32 be separated from a first mixture of HFC-125 and HFC-32, methylene chloride can be employed as an extractant in an extractive distillation-process that allows for efficient separation of the HFC-125 from the HFC-32. The HFC-32 may subsequently be separated from the methylene chloride by any number of known techniques, including simple distillation. The specific conditions which can be used for practicing the invention also depend upon a number of parameters such as the diameter of the column, feed points, number of separation stages in the column, among others. The operating pressure of the extractive distillation system may range from about 103.4 to 2413.2 kPa (15 to 350 psia), normally about 344.8 to 2068.4 kPa (50 to 300 psia). Typically, an increase in the methylene chloride feed rate relative to the HFC-125/HFC-32 feed rate causes an increase in the purity of the separated HFC-125 relative to the HFC-32. Normally, increasing the reflux results in an decreased methylene chloride in the overhead distillate. The temperature of the condenser, which is located adjacent to the top of the column, is normally sufficient to substantially condense the HFC-125.

Certain aspects of the invention are demonstrated by the following Examples. In the following Examples and Comparative Examples, "ppm" and "ppmw" are the parts-per-million-by-weight concentration of a compound in the identified stream.

In the following examples and comparative examples, "kph" and "kg/hr" are the kilograms-per-hour flowrate of the designated compound or stream. In parentheses following the kilograms-per-hour values are the equivalent values in the units of "pph" or "lbs/hour", the pounds-per-hour flowrate of the designated compound or stream.

Comparative Example 1

In this Comparative Example, a fresh feed stream consisting of HFC-32 and HFC-125 is fed to a first distillation column operated to separate the HFC-32 from the HFC-125. The column has 92 stages assumed to operate at 100% efficiency, with the condenser designated as stage 1. The column is 152,4 cm (60 inches) in diameter. The fresh feed stream contains equal weights of HFC-32 and HFC-125 and is fed onto stage 40 at -10 °C. The distillate from a second column that is fed back to this first column as a recycle feed stream also contains HFC-32 and HFC-125, and is also fed onto stage 40 of the first column. The condenser is operating at 24.1 °C., the reboiler is operating at 32.5 °C, and the column is operating at 1551,3 kPa (225 psig) pressure.

The distillate from the first column is then fed as the feed to a second column. This second column has 92 stages assumed to operate at 100% efficiency, with the condenser designated at stage 1. This second column is 221 cm (87 inches) in diameter. The feed stream is fed onto stage 40. The condenser is operating at -46.0 °C, the reboiler is operating at -43.7 °C, and the column is operating at 34,5 kPa (5 psig).

The results of these distillations are shown in Table 1.

TABLE 1

| | First Column Flow in Kgs Per Hour (Lbs per Hour) | | | | |
|---|---|---|---|---|---|
| | Column Fresh Feed | Recycle Feed | Column Reflux | Column Distillate | Column Bottoms |
| HFC-125 | 938.94 (2070.00) | 732.34 (1614.53) | 8443.62 (18615.00) | 733.28 (1616.61) | 937.99 (2067.92) |
| HFC-32 | 938.94 (2070.00) | 1873.42 (4130.18) | (71380.00) | 2811.95 (6199.28) | 0.41 (0.90) |

TABLE 1   (continued)

| | Second Column Flow in Kgs Per Hour (Lbs per Hour) | | | | |
|---|---|---|---|---|---|
| | Column Feed | Column Recycle | Column Distillate | Column Bottoms | |
| HFC-125 | 733.28 (1616.61) | 11474.07 (25296.00) | 732.34 (1614.53) | 0.94 (2.08) | |
| HFC-32 | 2811.95 (6199.28) | 29349.24 (64704.00) | 1873.42 (4130.18) | 938.53 (2069.10) | |

This Comparative Example shows that even with extremely large columns with many stages and an extremely high rate of reflux, a high efficiency of separation of the HFC-32 and HFC-125 cannot be obtained in a single column. The separation efficiency in a single column is limited by the existence of the HFC-125/HFC-32 azeotrope.

Example 1

In this Example, a feed stream consisting of approximately equal weights of HFC-32 and HFC-125 are fed to a first distillation column having 62 stages assumed to operate at 100 % efficiency, with the condenser designated as stage 1. The column is 48.26 cm (19 inches) in diameter. The column is operated at 413.7 kPa (60 psig), with the condenser operating at -8.1 degrees C, and the reboiler operating at 50.1 degrees C. The feed enters the column at -10 degrees C and is fed into the column onto stage 38. Methylene chloride from the bottoms ofa second column is fed into the first column as an extractant onto stage 12 at -5 degrees C.
The bottoms stream from the first column is then fed to the second distillation column, which has 24 stages assumed to operate at 100 % efficiency, with the condenser designated as stage 1. The second column is 23 inches in diameter. The column is operated at 482.7 kPa (70 psig), with the condenser operating at -9.9 °C, and the reboiler operating at 100 °C. The feed is fed into the column onto stage 12. The reflux flow in this example is 906 Kg/h (2000 pph).
The results of this distillation are shown in Table 2.

TABLE 2

| | First Column Flow in Kgs Per Hour (Lbs per Hour) | | | | |
|---|---|---|---|---|---|
| | Column Feeds | | Column Reflux | Column Distillate | Column Bottoms |
| | Stage 38 | Stage 12 | | | |
| HFC-125 | 938.94 (2070.00) | 0 | 1813.33 (3997.70) | 938.48 (2069) | 0.45 (1.00) |
| HFC-32 | 938.94 (2070.00) | 0 | 0.17 (0.37) | 0.086 (0.19) | 938.85 (2069.81) |
| $CH_2Cl_2$ | 0 | 18143.69 (400000.00) | <0.004 (<0.01) | <0.004 (<0.01) | 18143.69 (40000.00) |
| | Second Column Flow in Kgs Per Hour (Lbs per Hour) | | | | |
| | Column Feed | Column Recycle | Column Distillate | Column Bottoms | |
| HFC-125 | 0.45 (1.00) | 0.45 (1.00) | 0.45 (1.00) | <0.004 (<0.01) | |
| HFC-32 | 938.85 (2069.81) | 906.65 (1998.83) | 938.85 (2069.81) | <0.004 (<0.01) | |
| $CH_2Cl_2$ | 18143.69 (400000.00) | <0.004 (<0.01) | <0.004 (<0.01) | 18143.69 (400000.00) | |

In this Example, the same high degree separation of both the HFC-125 and HFC-32 which in Comparative Example 1 required two columns is obtained in this Example via a single extractive distillation column. Separating the HFC-32 exiting the bottoms of the first column from the methylene chloride extractant is easily effected by the second distillation..Combined, the pair of columns in this Example required to obtain the two separate and high purity HFC-125 and HFC-32 product streams from the initial HFC-125/HFC-32 fresh feed stream have significantly smaller diameters, fewer stages, and lower reflux flows than the pair of columns required in Comparative Example 1. Compared to Comparative Example 1, this translates into significantly lower investment and energy costs to effect the separation.
The following examples illustrate the results obtained by co-feeding precursors of HFC-32 and HFC-125 with HF in a catalytic reactor under a variety of reaction conditions:

Example 2

The catalyst of U.S. Patent No. 5,334,787, hereby incorporated by reference, was prepared and pre-treated with hydrogen fluoride (HF) as disclosed in that patent. Mixtures comprising hydrogen fluoride, methylene chloride, and HCFC-124 were fed over the catalyst at atmospheric pressure, at the temperature and contact time listed below in Table 3, and with results as noted:

Table 3

| Organic Mole % HFC-32, HFC-125, CFC-115 vs. Operating Condition | | | | | | | |
|---|---|---|---|---|---|---|---|
| Contact Time (Sec) | HF/Tot.Org. in Feed (Mole Ratio) | HF/HCC-30 in Feed (Mole Ratio) | HF/HCFC-124 in Feed (Mole Ratio) | Temp (deg.C) | Mole %32 | Mole %125 | Mole %115 |
| 10 | 3.1 | 4.8 | 8.5 | 301 | 32.1 | 12.4 | <0.05 |
| 10 | 3.1 | 7.2 | 5.4 | 301 | 25.1 | 21.2 | 0.1 |
| 31 | 3.4 | 4.9 | 11.0 | 301 | 32.1 | 15.8 | 0.2 |
| 31 | 3.4 | 7.3 | 6.4 | 301 | 29.8 | 13.4 | 0.1 |
| 10 | 6.5 | 9.9 | 18.6 | 301 | 43.8 | 13.9 | <0.05 |
| 10 | 6.4 | 14.3 | 11.5 | 301 | 31.7 | 21.6 | <0.05 |
| 30 | 6.5 | 8.3 | 30.3 | 301 | 48.9 | 17.6 | 0.1 |
| 31 | 8.0 | 16.6 | 15.5 | 301 | 39.3 | 27.5 | 0.2 |
| 20 | 4.7 | 7.9 | 11.8 | 301 | 36.0 | 21.5 | 0.1 |
| 20 | 5.0 | 8.6 | 11.8 | 327 | 42.8 | 17.4 | 0.2 |
| 20 | 4.7 | 7.9 | 11.8 | 327 | 30.0 | 37.4 | 0.3 |
| 20 | 4.8 | 6.8 | 15.7 | 327 | 34.8 | 33.4 | 0.3 |
| 20 | 5.3 | 11.9 | 9.4 | 327 | 25.6 | 50.0 | 0.4 |
| 10 | 4.7 | 8.6 | 10.3 | 327 | 32.8 | 31.4 | 0.2 |
| 30 | 5.0 | 8.0 | 13.6 | 327 | 51.0 | 20.9 | 0.5 |
| 20 | 3.1 | 5.5 | 7.3 | 327 | 24.0 | 32.5 | 0.4 |
| 20 | 7.2 | 12.4 | 17.0 | 327 | 34.2 | 29.0 | 0.3 |
| 20 | 4.7 | 7.9 | 11.8 | 327 | 33.7 | 27.5 | 0.3 |
| 10 | 3.1 | 4.8 | 8.5 | 351 | 26.8 | 27.9 | 0.6 |
| 10 | 3.1 | 7.2 | 5.4 | 351 | 18.8 | 44.3 | 0.7 |
| 31 | 3.4 | 4.9 | 11.0 | 351 | 26.0 | 14.8 | 0.9 |
| 31 | 3.4 | 7.3 | 6.4 | 351 | 20.5 | 44.0 | 1.5 |
| 10 | 6.5 | 9.9 | 18.6 | 351 | 38.8 | 31.8 | 0.3 |
| 10 | 6.4 | 14.3 | 11.5 | 351 | 25.8 | 50.6 | 0.4 |

Where:
"Contact Time" is the contact time in the reactor, in seconds.
"Temp" is the temperature the reaction is being run, in degrees C.
"HF/Tot.Org." is the HF / Total Organic molar feed ratio to the reactor.
"HF/HCC-30" is the HF / HCC-30 molar feed ratio to the reactor.
"HF/HCFC-124" is the HF / HCFC-124 molar feed ratio to the reactor.
"%32" is the organic mole % HFC-32 in the reactor off-gas.
"%125" is the organic mole % HFC-125 in the reactor off-gas.
"%115" is the organic mole % CFC-115 in the reactor off-gas.

This Example shows how a variety of HFC-32 and HFC-125 molar ratios and productivities may be obtained by adjusting feed ratios and operating conditions.

Example 3

The catalyst described in U.S. Patent Application Serial No. 08/146,334 (Attorney Docket No. CR-9436) was prepared and pre-treated with hydrogen fluoride (HF) as disclosed in that patent application. Mixtures comprising hydrogen fluoride, methylene chloride, and HCFC-124 were fed over the catalyst at atmospheric pressure, at the temperature and contact time listed below in Table 4, and with results as noted in Table 4:

Table 4

| Organic Mole % HFC-32, HFC-125 vs. Operating Conditions | | | | | |
|---|---|---|---|---|---|
| Contact Time (s) | Temp (°C) | HF/Tot.Org. | %HCC-30 | %HFC-32 | %HFC-125 |
| 26 | 327 | 5.2 | 58 | 40 | 27 |
| 12 | 304 | 3.4 | 64 | 38 | 20 |
| 12 | 302 | 3.4 | 42 | 24 | 24 |
| 12 | 302 | 7.2 | 66 | 47 | 18 |
| 12 | 302 | 7.2 | 44 | 31 | 32 |
| 26 | 302 | 5.2 | 58 | 32 | 18 |
| 27 | 329 | 5.2 | 58 | 40 | 27 |
| 26 | 327 | 5.3 | 69 | 49 | 24 |
| 26 | 327 | 4.9 | 51 | 36 | 35 |
| Where:<br>"Contact Time" is the contact time in the reactor, in seconds.<br>"Temp" is the temperature the reaction is being run, in degrees C.<br>"HF/Tot.Org." is the HF / Total Organic molar feed ratio to the reactor.<br>"% HCC-30" is the organic mole % HCC-30 in the feed stream to the reactor.<br>"% HFC-32" is the organic mole % HFC-32 in the reactor off-gas.<br>"% HFC-125" is the organic mole % HFC-125 in the reactor off-gas. | | | | | |

This Example shows how a variety of HFC-32 and HFC-125 molar ratios and productivities may be obtained by adjusting feed ratios and operating conditions.

In general, this chemistry as shown in Example 2 and Example 3 may be operated at temperatures that range from about 175 to 400 °C; normally from about 250 to 350 °C. This chemistry may also be operated by employing an HF/total organic molar feed ratio of from about 2/1 to 10/1; normally from about 4/1 to 8/1, and may be operated from atmospheric pressure up to 1379.1 kPa (200 psig). The chemistry may be used to obtain off-gas compositions containing from about 5 to 70 organic mole % HFC-125 and from 5 to 70 organic mole % HFC-32. The catalyst of Example 3 is desirable for this chemistry because the catalyst of Example 3 is particularly resistant to deactivation in this chemistry, and the catalyst of Example 3 can subsequently be regenerated after any activity loss. The catalyst of Example 3 may bereactivated, regaining its initial, or, in some cases, obtaining greater than its initial activity, by, for example, using the procedure described in US Patent No. 4,155,881.

**Claims**

1.    A process for separating HFC-32 and HFC-125 from a first mixture by using an extractant comprising methylene chloride, the process comprising the steps of: adding the extractant to the first mixture in order to form a second mixture, separating HFC-32 and HFC-125 in the second mixture by extractively distilling the second mixture in an extractive distillation zone of a distillation column and thereby recovering HFC-125 as an overhead product of the column and HFC-32 from the column bottom.

**Patentansprüche**

1.    Verfahren zum Trennen von HFC-32 und HFC-125 aus einer ersten Mischung unter Verwendung eines Extraktionsmittels, das Methylenchlorid umfaßt, wobei das Verfahren die folgenden Schritte umfaßt: Zugabe des Extraktionsmittels zur ersten Mischung, um eine zweite Mischung zu bilden, Trennen des HFC-32 und HFC-125 in der zweiten Mischung durch extraktive Destillierung der zweiten Mischung in der Extraktionsdestillationszone einer Destillationskolonne und dadurch Gewinnen von HFC-125 als Kopfprodukt der Kolonne und HFC-32 aus dem Kolonnensumpf.

**Revendications**

1. Procédé pour la séparation de HFC-32 et HFC-125 à partir d'un premier mélange en utilisant un solvant d'extraction comprenant du chlorure de méthylène, le procédé comprenant les étapes suivantes : addition du solvant d'extraction au premier mélange pour former un second mélange, séparation de HFC-32 et HFC-125 dans le second mélange par distillation extractive du second mélange dans une zone de distillation extractive d'une colonne de distillation et de cette façon récupération de HFC-125 comme distillat de tête de la colonne et de HFC-32 comme produit de bas de colonne.

FIG. 1